# FASCICULE DE BREVET EUROPEEN

(11) **EP 1 335 752 B1**
(45) Date de publication et mention de la délivrance du brevet: **08.11.2006**
(21) Numéro de dépôt: 01996395.8
(22) Date de dépôt: 16.11.2001
(51) Int. Cl.: A61L 2/26

(54) **EMBALLAGES MULTIFONCTIONNELS POUR PRODUITS STERILISES OU DESTINES A ETRE STERILISES**
MEHRZWECK-VERPACKUNGEN FÜR STERILISIERTE PRODUKTE ODER ZU STERILISIERENDE PRODUKTE
MULTIPURPOSE PACKAGES FOR STERILISED PRODUCTS OR PRODUCTS TO BE STERILISED

(30) Priorité: 20.11.2000 FR 0014977
(43) Date de publication de la demande: 20.08.2003
(73) Titulaire: Becton Dickinson France, 38800 Le Pont de Claix (FR)
(72) Inventeur: JANSEN, Hubert, D-35041 Marburg-Michelbach (FR); PORRET, Jean-Yves, F-38610 GIERES (FR)
(74) Mandataire: Brédeville, Odile Marie
(86) Numéro de dépôt international: PCT/FR2001/003612
(87) Numéro de publication internationale: WO 2002/040063

(56) Documents cités:
- WO-A-91/11204
- DE-A- 2 439 900
- US-A- 5 496 302
- US-A- 6 085 492

## Description

La présente invention se rapporte au domaine des emballages stériles ou stérilisés, et plus particulièrement aux emballages destinés à transporter des produits stérilisés ou destinés à être stérilisés.

Les conditions de stérilité dans lesquelles doivent se dérouler certaines étapes de manipulation ou de transport des produits ou ustensiles destinés à un usage médical sont très contraignantes et en particulier dans l'industrie pharmaceutique. Il est donc de grande importance de réaliser des emballages compatibles avec de telles exigences.

Dans la suite de la description, il sera fait mention d'un certain nombre d'expressions qu'il convient de définir ci-après.

Par l'expression "sélectivement étanche", telle qu'utilisée dans la présente description ainsi que dans les revendications, on entend que le matériau est conçu en termes de structure de manière à contrôler tout échange de l'intérieur de l'emballage avec son environnement extérieur. Ceci signifie entre autres que l'emballage est étanche individuellement où en combinaison à la contamination par des microorganismes, bactéries et/ou un matériau biologiquement actif, susceptible de venir en contact avec l'emballage lors de sa manipulation, tout en restant perméable à un gaz de stérilisation ou de décontamination par exemple du type oxyde d'éthylène ou fluide décontaminant ou stérilisant à haute température, par exemple de la vapeur d'eau jusqu'à 130°C, plus généralement 120° à 127°C et préférentiellement entre 121° et 123°C.

Par "haute température", il faut entendre également et plus généralement des températures proches des températures de déformation des matériaux plastiques en présence.

Par "matériau plastique", il faut entendre tout matériau choisi dans des familles des polymères tels que les styréniques, acryliques, polysulfones, polycarbonates, polyesters, polyoléfines, etc..., en incluant les copolymères, combinaisons et alliages de polymères. Le "matériau plastique" est par exemple du polystyrène, du polyéthylène ou du polypropylène.

Il sera également fait mention d'un "écran vis-à-vis d'une irradiation électronique", lequel doit être compris comme incluant un matériau susceptible de réfléchir et/ou d'absorber partiellement ou en totalité l'énergie cinétique des électrons issus d'un faisceau et par conséquent de ralentir, voire d'empêcher ces derniers de traverser ledit matériau.

L'expression "écran vis-à-vis d'un rayonnement lumineux" doit être comprise comme définissant un matériau permettant de réfléchir, atténuer ou empêcher un rayonnement lumineux, par exemple pulsé ou ultraviolet, de le traverser.

Les termes "transparent" et "opaque" sont à considérer vis-à-vis d'une irradiation électronique d'une part et vis-à-vis d'un rayonnement lumineux d'autre part. Un matériau est transparent s'il est susceptible d'être traversé par un rayonnement lumineux ou par des électrons lorsqu'il est soumis à une irradiation électronique. Un matériau est par conséquent opaque vis-à-vis d'une irradiation électronique ou d'un rayonnement lumineux s'il permet de réfléchir, atténuer et/ou absorber l'énergie cinétique des électrons ou s'il empêche un rayonnement lumineux de le traverser. L'opacité et/ou la transparence d'un matériau sont le plus souvent déterminés à partir de paramètres tels que, épaisseur, densité, coefficient de réflexion ou d'atténuation.

On connaît des emballages pour produits stériles ou destinés à être stérilisés par un gaz du type oxyde d'éthylène, comportant une boîte en matière plastique et un couvercle réalisé en matériau sélectivement étanche, permettant de sceller par une zone de scellage étanche ladite boîte.

Certains emballages comme ceux utilisés pour transporter des seringues avant leur remplissage par un produit actif ou médicament, sont actuellement transportés dans des boîtiers en plastique, par exemple du polystyrène, recouvertes d'une feuille de couverture réalisée avec un matériau sélectivement étanche. Ce dernier est par exemple une feuille à base de filaments de PEHD (polyéthylène haute densité) ou autre polymère, liés notamment par l'intermédiaire de chaleur et de pression. Un tel produit est par exemple commercialisé sous la marque TYVEK^{®}.

Des produits destinés à être stérilisés sont ainsi disposés à l'intérieur d'une boîte, laquelle est ensuite scellée par l'intermédiaire de la feuille sélectivement étanche. Un fluide de stérilisation pénètre ensuite dans la boîte à travers la feuille de matériau sélectivement étanche. La boîte contenant les produits stérilisés est alors disposée dans un sac de protection pour la transporter. Un autre mode consiste à introduire d'abord la boîte dans le sac pourvu d'une zone sélectivement étanche et procéder ensuite à la stérilisation.

A titre d'exemple, une telle boîte ou emballage peut contenir des seringues destinées à être remplies par un médicament dans une salle stérile ou à environnement contrôlé. Avant le remplissage desdites seringues, il est nécessaire d'ouvrir le sac de protection et de décontaminer l'emballage éventuellement contaminé, avant son introduction par exemple dans une salle stérile. Une telle décontamination peut être obtenue à l'aide d'un faisceau d'électrons développant une énergie suffisante pour présenter après la traversée de la feuille de couverture (matériau sélectivement étanche), une dose d'irradiation de 25 kGy. Cela permet de considérer que le matériau sélectivement étanche a été décontaminé dans toute son épaisseur, et en particulier dans la zone scellée à l'interface de la boîte et dudit matériau sélectivement étanche.

Ce type de décontamination par l'intermédiaire d'un faisceau d'électrons peut présenter cependant des inconvénients. En effet, les électrons traversant éventuellement la feuille du matériau sélectivement étanche risquent d'une part de modifier ou d'altérer le matériau constitutif des seringues ou produits disposés dans la boîte, par exemple du verre, et d'autre part de générer avec l'oxygène de l'air contenu dans ladite boîte, de l'ozone. Ce dernier peut altérer des éléments ou des pièces en caoutchouc comme par exemple des capuchons d'aiguilles montés sur des seringues ou polluer l'atmosphère. Le remplissage des seringues avec un médicament peut également être déconseillé dans un milieu contenant de l'ozone.

L'utilisation d'une décontamination à l'aide d'un rayonnement lumineux n'est par ailleurs pas indiquée car ledit rayonnement lumineux n'est pas en mesure de traverser la feuille de matériau sélectivement étanche lorsque celle ci est opaque à la lumière, ce qui est généralement le cas, et par conséquent d'atteindre une zone localisée à l'interface du couvercle et de la boîte. Cette zone contenant par exemple une couche de colle peut présenter des irrégularités et il est par conséquent indispensable d'atteindre ces irrégularités éventuellement contaminées, avec un moyen de décontamination.

En outre, il est à noter qu'une stérilisation à l'aide d'un fluide stérilisant ou décontaminant à haute température, par exemple de la vapeur d'eau à 121°C, provoque des contraintes dans les matériaux en présence, en l'occurrence le matériau appelé TYVEK^{®} et celui de la boîte ; ce qui génère compte tenu des dimensions relativement importantes des boîtes et emballages, des tensions sur le moyen de liaison entre ladite feuille de matériau sélectivement étanche et le bord périphérique de la boîte. Ces tensions peuvent provoquer des déformations de la boîte et/ou une désolidarisation entre la feuille de matériau sélectivement étanche et ladite boîte.

D'autres agents de stérilisation par voie gazeuse sont par exemple des vapeurs ou plasmas de peroxyde d'hydrogène, de formaldéhyde, de glutaraldehyde, d'acide peracetique, de dioxyde de chlore, d'ozone, etc.

Les inconvénients de ces agents de stérilisation sont liés à la difficulté de maîtrise desdites vapeurs ou plasmas, aux caractères toxiques et agressifs de certains agents et/ou résidus, et à leur incompatibilité avec certains matériaux.

On connaît également la stérilisation par rayonnements électromagnétiques, par exemple par micro-ondes. Le même type d'emballage que celui utilisé par une stérilisation par voie gazeuse est alors utilisé, et ce afin de permettre des échanges gazeux entre l'atmosphère extérieure et le volume intérieur de la boîte. Ces échanges sont nécessaires du fait des dilatations thermiques de l'atmosphère intérieure de la boîte, liés à des variations et élévations de température.

Le document US-A-5 496 302 décrit un dispositif comprenant un emballage en matériau sélectivement étanche et connecté à un dispositif de fourniture. Lors de la stérilisation du dispositif de fourniture par rayonnement l'emballage est protégé par un écran.

L'objet de la présente invention vise à réaliser un emballage pour produits destinés à être stérilisés, par tout type de stérilisation, et en particulier une stérilisation à l'oxyde d'éthylène (ETO) ou à l'aide d'un fluide stérilisant à haute température, ledit emballage étant également susceptible d'être décontaminé extérieurement par l'intermédiaire de divers types de décontamination, et en particulier par l'intermédiaire d'un faisceau électronique, d'une lumière pulsée ou un rayonnement ultraviolet.

Un autre objet de la présente invention vise à réaliser un emballage pour produits destinés à être stérilisés, lequel est susceptible d'indiquer quel type de stérilisation et de décontamination il a subi.

Un objet additionnel de la présente invention vise à réaliser un emballage scellant de façon étanche une boîte contenant des produits destinés à être stérilisés à l'aide d'un fluide de stérilisation du type vapeur ou oxyde d'éthylène, en réduisant au mieux le temps nécessaire à une telle opération de stérilisation.

Les objets de la présente invention sont atteints à l'aide d'un emballage pour produits stériles ou destinés à être stérilisés comportant une boîte en matière plastique et un couvercle fixé sur la boîte de manière à sceller cette dernière de façon sélectivement étanche.

Selon l'invention, le couvercle comprend
- une feuille de couverture en matériau plastique, transparente pour une irradiation électronique et pour un rayonnement lumineux ;
- au moins une fenêtre ménagée dans la feuille de couverture ;
- au moins une feuille d'un matériau sélectivement étanche, solidaire de ladite feuille de couverture et obturant la fenêtre ;
- et un écran opaque pour au moins une irradiation électronique traversant la feuille de couverture ou le matériau sélectivement étanche, ledit écran s'étendant à l'intérieur de l'emballage, au voisinage de la feuille de couverture, de manière à permettre la pénétration d'un gaz de stérilisation, par exemple oxyde d'éthylène ou vapeur d'eau, à travers le matériau sélectivement étanche.

Selon un mode de réalisation de l'emballage conforme à l'invention, le couvercle comprend des indicateurs d'utilisation permettant d'identifier et d'indiquer la nature de la stérilisation et de la décontamination auxquels il a été soumis.

Selon un mode de réalisation de l'emballage conforme à l'invention, le matériau sélectivement étanche présente une zone de liaison périphérique sur laquelle est répartie de façon discrète ou continue, une colle compatible avec une stérilisation à haute température et une zone centrale restant dépourvue de colle.

L'emballage conforme à l'invention présente l'avantage qu'il est utilisable, avec plusieurs procédés de stérilisation et de décontamination. L'emballage peut être utilisé tel quel ou avec des aménagements prédéfinis. Il n'est donc pas nécessaire de concevoir un produit particulier compatible avec une méthode de stérilisation et une décontamination particulière utilisée.

D'autres caractéristiques et avantages ressortiront également de la description détaillée figurant ci-après, donnée à titre d'exemple, par référence au dessin annexé, dans lequel :
- la figure 1 représente une vue en perspective partiellement découpée d'un exemple d'emballage conforme à l'invention ;
- la figure 2 montre l'exemple de réalisation de la figure 1 selon une coupe suivant la direction II-II de la figure 1 ;
- la figure 3 représente une vue de dessus de l'exemple de réalisation de la figure 1 ;
- la figure 4 représente un autre mode de réalisation de l'emballage conforme à l'invention.

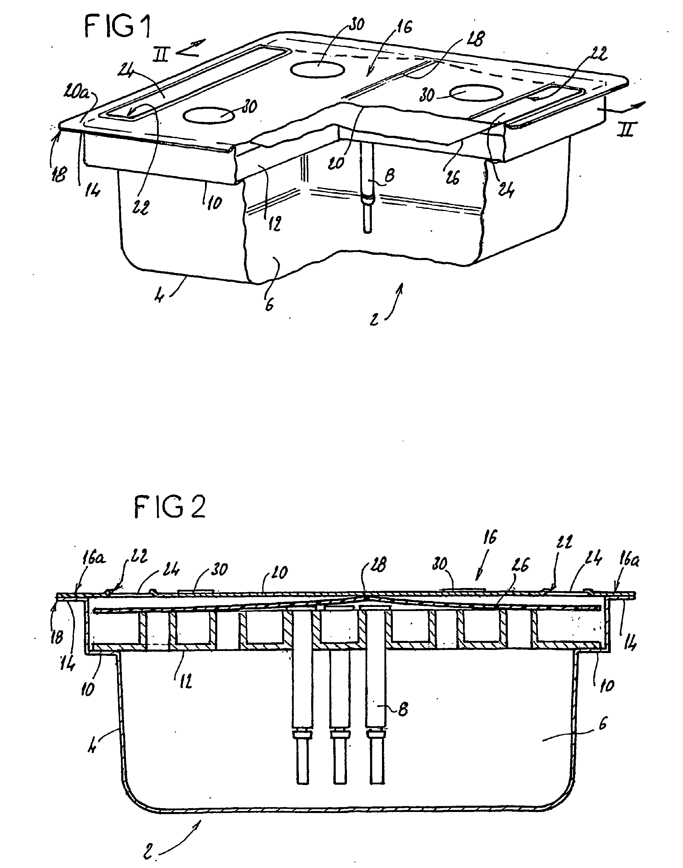

L'emballage 2 conforme à l'invention comporte une boîte 4 (partiellement découpé sur la figure 1) délimitant un volume interne 6, destiné à contenir une charge à stériliser. Cette charge est par exemple constituée de seringues 8. La boîte 4 présente intérieurement à cet effet un premier bord périphérique 10 s'étendant vers l'extérieur de la boîte 4 et destiné à réaliser un appui pour un support 12 pour les seringues 8. Ce support 12 est par exemple constitué d'une plaque sur laquelle sont ménagées des cheminées ou des orifices, traversés par les seringues 8.

La boîte 4 présente également un bord périphérique supérieur 14 sensiblement horizontal et s'étendant vers l'extérieur de ladite boîte 4. Cette dernière est par exemple réalisée dans un matériau plastique du type polystyrène ou autre polymère. Le matériau constitutif de la boîte 4 peut être opaque, transparent ou semi-transparent vis-à-vis d'un rayonnement lumineux par exemple visible ou du type ultraviolet.

L'emballage 2 comporte également un couvercle 16 fixé sur la boîte 4 de manière à sceller cette dernière par une zone de scellage étanche. Le couvercle 16 est par exemple collé avec son bord périphérique 16a sur le bord périphérique supérieur 14 de la boîte 4. Une couche de colle 18 permet de réaliser l'interface entre le bord périphérique 16a et le bord périphérique supérieur 14. La couche de colle, par exemple du type "hot melt" 18 est choisie parmi des colles résistant à des hautes températures par exemple de l'ordre de 121°C correspondant à une stérilisation vapeur d'eau.

Le couvercle 16 comprend une feuille de couverture 20 en matériau plastique permettant de sceller la boîte 4. La feuille de couverture 20 est transparente pour une irradiation électronique ainsi que pour un rayonnement lumineux par exemple ultraviolet. Les caractéristiques opacité/densité/épaisseur de la feuille de couverture 20 sont donc choisies en conséquence. La feuille de couverture 20 présente au moins une fenêtre 22, obtenue par exemple par tout moyen connu, notamment par découpage. La feuille de couverture 20 représentée aux figures comporte deux fenêtres 22. Chaque fenêtre 22 présente par exemple une forme longitudinale et s'étend au voisinage des bords périphériques supérieurs 14 de la boîte 4

Le couvercle 16 comprend également au moins une feuille 24 d'un matériau sélectivement étanche solidaire de la feuille de couverture 16 et obturant chacune des fenêtres 22.

Le matériau sélectivement étanche 24 est par exemple un matériau à base de filaments PEHD (polyéthylène haute densité) ou autres polymères, liés entre autres par l'intermédiaire de chaleur et de pression. Les filaments sont agglomérés de manière à former une structure microporeuse, imperméable notamment aux microorganismes ou autres bactéries. Selon un exemple de réalisation, le matériau sélectivement étanche 24 comprend du TYVEK^{®}. La feuille 24 de matériau sélectivement étanche est, avec sa périphérie 24a, collée sur ou préférentiellement sous la feuille de couverture 20. Le matériau sélectivement étanche peut également être constitué de fibres naturelles telles que des fibres végétales, par exemple de cellulose, compatibles avec une stérilisation à haute température.

Le couvercle 16 est associé par ailleurs à un écran 26 opaque pour au moins une irradiation électronique traversant la feuille de couverture 20. Cet écran 26 s'étend à l'intérieur de l'emballage 2 au voisinage de la feuille de couverture 20 de manière à permettre la pénétration d'un gaz de stérilisation, par exemple de l'oxyde d'éthylène (ETO) ou de la vapeur, à travers le matériau sélectivement étanche.

L'écran 26 comprend par exemple une feuille métallique souple. L'écran 26 peut ainsi être constitué d'une feuille souple d'aluminium.

Dans les exemples de réalisation montrés plus particulièrement aux figures 1 et 2, l'écran 26 est fixé sous la feuille de couverture 20 suivant une ligne de fixation 28, des points de fixation ou une zone de fixation, à proximité par exemple du centre de ladite feuille de couverture 20. Tous moyens de fixation ou de liaison connus peuvent être envisagés pour réaliser cette ligne de fixation 28. Cette dernière peut être obtenue par exemple par collage, soudure ou gaufrage.

Selon un autre mode de réalisation conforme à l'invention, l'écran 26 comporte par exemple un assemblage de deux feuilles de matériau sélectivement étanche. Un tel assemblage peut également être fixé sur la feuille de couverture 20 suivant la ligne de fixation 28. La position du bord périphérique 10 ainsi que les dimensions du support 12 sont choisies de manière à ce que l'écran 26, lequel repose de part et d'autre de la ligne de fixation 28 sur ledit support 12, s'étend à proximité et sous la feuille de couverture 20. On réduit ainsi au maximum l'écart entre l'écran 26 et la feuille de couverture 20, tout en conservant un passage pour un gaz de stérilisation.

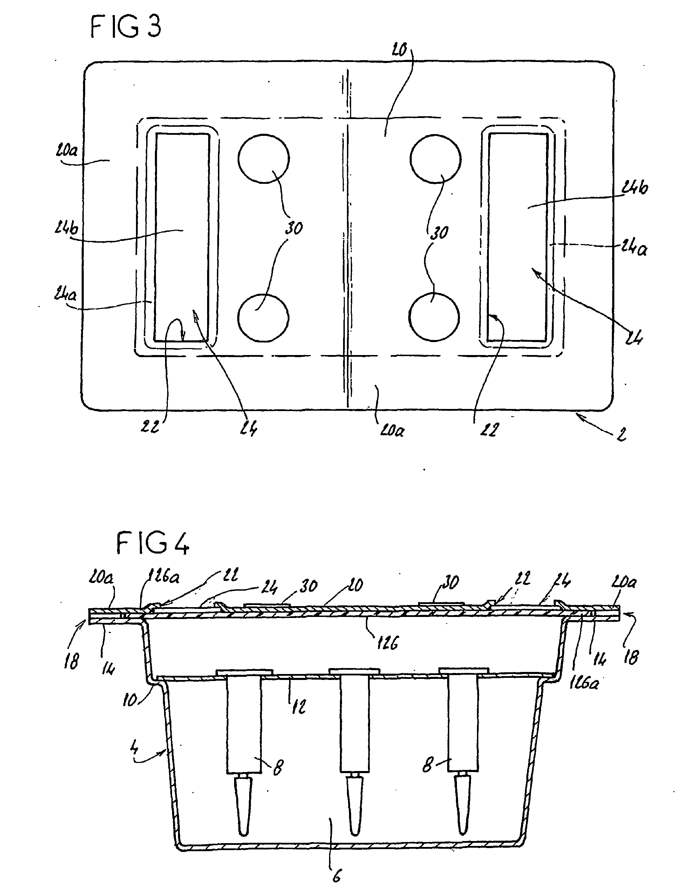

Un autre exemple de réalisation représenté à la figure 4 montre l'écran 126 par exemple constitué d'un assemblage de deux feuilles de matériau sélectivement étanche. Dans cet exemple de réalisation, l'écran 126 s'étend sous la feuille de couverture 20. L'écran 126 présente également des dimensions permettant à sa partie périphérique 126a d'être prise en sandwich entre la feuille de couverture 20 et le bord périphérique supérieur 14 de la boîte 4. Le scellage de la boîte 4 par la feuille de couverture 20 est obtenu par la couche de colle 18 réalisant l'interface entre ladite feuille de couverture 20 et ledit bord périphérique supérieur 14, et l'écran 126 peut être solidarisé ou non avec la feuille de couverture 20 et/ou avec le bord périphérique supérieur 14. Selon un autre mode de réalisation, l'écran 126 est étanche. Dans ce cas de figure l'écran 126 présente également des orifices en communication fluidique avec au moins une fenêtre 22.

Le couvercle 16 de l'emballage 2 conforme à l'invention comprend également des indicateurs d'utilisation 30 permettant d'identifier et d'indiquer la nature de stérilisation et de décontamination à laquelle ledit emballage 2 a été soumis. Les indicateurs d'utilisation 30, connus en tant que tels, sont par exemple des indicateurs qui modifient leur couleur en fonction du traitement subi. Le manipulateur des emballages 2 sait donc en permanence à quel type de stérilisation les seringues 8 ont été soumises, et à quel type de décontamination l'emballage 2 a été soumis.

La feuille de couverture 20 est transparente vis-à-vis d'un rayonnement électronique et vis-à-vis d'un rayonnement lumineux. La transparence de la feuille de couverture 20 par rapport à une irradiation électronique et par rapport à un rayonnement lumineux du type lumière pulsée ou rayonnement ultraviolet permet de décontaminer une zone située à l'interface entre ladite feuille de couverture 20 et le bord périphérique supérieur 14. Des irrégularités dans la couche de colle 18, éventuellement contaminées peuvent ainsi être décontaminées par un faisceau d'électrons ou un rayonnement lumineux tel que mentionné précédemment.

La boîte 4 est par exemple réalisée avec une matière plastique transparente au rayonnement lumineux. La transparence par rapport à un rayonnement lumineux visible permet d'identifier et de contrôler en continu lors d'une éventuelle décontamination, le contenu de l'emballage 2. Le matériau constitutif de la boîte 4 présente par exemple un couple densité-épaisseur tel qu'il arrête les électrons issus d'un faisceau d'électrons d'irradiation. Ceci permet d'éviter une altération des produits ou de la charge contenue dans l'emballage 2, en l'occurrence les seringues 8, ainsi que la génération d'ozone avec l'air.

Le matériau sélectivement étanche présente de préférence une zone de liaison périphérique 24a sur laquelle est répartie de façon continue, une colle compatible avec une stérilisation à haute température, et une zone centrale 24b restant dépourvue de colle. L'absence de colle dans cette zone centrale 24b facilite et accélère le passage d'un gaz de stérilisation du type ETO ou vapeur d'eau à travers le matériau sélectivement étanche 24. Les durées nécessaires à la stérilisation d'un emballage 2 peuvent ainsi être réduites.

L'écran 126 s'étend sous chaque fenêtre 22 de manière à réaliser une zone perméable à un gaz de stérilisation du type ETO ou vapeur d'eau. Ce gaz traverse ainsi trois couches d'un matériau sélectivement étanche. Une telle épaisseur est opaque d'une part vis-à-vis d'un rayonnement lumineux, et d'autre part vis-à-vis de certaines irradiations électroniques. Une dose d'irradiation par exemple de 25 kGy après la traversée du matériau sélectivement étanche obturant la fenêtre 22, peut ainsi être absorbée par l'écran 126. On évite ainsi une pénétration d'électrons dans le volume intérieur 6 et par conséquent la création d'ozone ou une altération des seringues 8.

Une telle dose d'irradiation électronique utilisée pour une décontamination est également absorbée par l'écran 26 (cf. figures 1 et 2) dont la densité et/ou l'épaisseur sont choisies en conséquence. Le couple densité/épaisseur de la feuille de couverture 20 est également choisi pour rendre ladite feuille de couverture 20 transparente vis-à-vis d'une irradiation électronique et en particulier sa partie périphérique 20a fixée ou collée sur le bord périphérique supérieur 14. Un rayonnement lumineux, par exemple ultraviolet peut ainsi décontaminer l'interface de liaison entre ladite feuille de couverture 20 et le bord périphérique supérieur 14. L'écran 26 (cf. figure 2) présente de chaque côté de la ligne de fixation 28, des parties qui ne sont pas liées intimement à la feuille de couverture 20 et en particulier au matériau sélectivement étanche, de manière à ne pas altérer le passage d'un gaz de stérilisation du type ETO ou vapeur. Ceci est particulièrement intéressant lorsque l'écran 26 est imperméable à un gaz de stérilisation comme c'est le cas par exemple pour une feuille d'aluminium.

Selon un autre mode de réalisation de l'emballage conforme à l'invention, il est également possible de remplacer pour certains matériaux, la couche de colle 18 par un thermoscellage.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Ainsi, l'écran 26 peut ne pas être relié du tout à la feuille de couverture et simplement reposer sur les produits contenus dans l'emballage, sur une pièce contenue dans l'emballage ou sur des appuis que celui-ci comprend intérieurement.

## Revendications

1. Emballage (2) pour produits stériles ou destinés à être stérilisés comportant une boîte (4) en matière plastique et un couvercle (16) fixé sur la boîte (4) de manière à sceller cette dernière par une zone de scellage étanche, le couvercle (16) comprenant :
- une feuille de couverture (20) en matériau plastique, transparente pour une irradiation électronique et pour un rayonnement lumineux ;
- au moins une fenêtre (22) ménagée dans la feuille de couverture (20),
- au moins une feuille (24) d'un matériau sélectivement étanche à la contamination par des microorganismes, bactéries et/ou un matériau biologiquement actif, tout en restant perméable à un gaz de stérilisation ou de décontamination, solidaire de ladite feuille de couverture (20) et obturant la fenêtre (22) ;
- et un écran (26, 126) opaque pour au moins une irradiation électronique traversant la feuille de couverture ou le matériau sélectivement étanche, ledit écran s'étendant à l'intérieur de l'emballage (2), au voisinage de la feuille de couverture (20), de manière à permettre la pénétration d'un gaz de stérilisation, par exemple oxyde d'éthylène ou vapeur d'eau, à travers le matériau sélectivement étanche.

2. Emballage (2) selon la revendication 1, **caractérisé en ce que** la (les) feuilles (24) de matériau sélectivement étanche est (sont) collée(s) sous la feuille de couverture (20).

3. Emballage (2) selon la revendication 1 ou 2, **caractérisé en ce que** le matériau sélectivement étanche est un matériau à base de filaments PEHD ou autre polymère, liés entre autres par l'intermédiaire de chaleur et de pression.

4. Emballage (2) selon l'une quelconque des revendications 1 à 3, **caractérisé en ce que** la fenêtre (22) s'étend au voisinage des bords périphériques supérieurs (14) de la boîte (4).

5. Emballage (2) selon l'une quelconque des revendications 1 à 4, **caractérisé en ce que** l'écran (26, 126) comporte un assemblage de deux feuilles de matériau sélectivement étanche.

6. Emballage (2) selon l'une quelconque des revendications 1 à 4, **caractérisé en ce que** l'écran (26, 126) comporte un matériau métallisé ou une feuille métallique souple.

7. Emballage (2) selon la revendication 6, **caractérisé en ce que** l'écran (26) est une feuille souple d'aluminium.

8. Emballage (2) selon l'une quelconque des revendications 1 à 7, **caractérisé en ce que** l'écran (26, 126) est fixé sous la feuille de couverture (20), suivant une ligne de fixation (28) ou des points de fixation.

9. Emballage (2) selon l'une quelconque des revendications 1 à 5, **caractérisé en ce que** l'écran (126) sélectivement étanche, s'étend sous la feuille de couverture (20), et présente des dimensions, permettant à sa partie périphérique (126a) d'être prise en sandwich entre la feuille de couverture (20) et le bord périphérique supérieur (14) de la boîte (4).

10. Emballage (2) selon l'une quelconque des revendications 1 à 7, **caractérisé en ce que** l'écran (126) étanche, s'étend sous la feuille de couverture (20) et présente des dimensions permettant à sa partie périphérique (126a) d'être prise en sandwich entre la feuille de couverture (20) et le bord périphérique supérieur (14) de la boîte (4) d'une part, et présente des orifices, en communication fluidique avec au moins une fenêtre (22) d'autre part.

11. Emballage (2) selon l'une quelconque des revendications 1 à 10, **caractérisé en ce que** le couvercle (16) comprend des indicateurs d'utilisation (30) permettant d'identifier et d'indiquer la nature de stérilisation et/ou de décontamination, auxquelles ledit emballage (2) a été soumis.

12. Emballage (2) selon l'une quelconque des revendications 1 à 11, **caractérisé en ce que** la boîte (4) est en matière plastique transparente aux rayonnements lumineux.

13. Emballage (2) selon l'une quelconque des revendications 1 à 12, **caractérisé en ce que** la feuille (24) de matériau sélectivement étanche présente une zone de liaison périphérique (24a) sur laquelle est répartie de façon continue une colle compatible avec une stérilisation à haute température, et une zone centrale (24b) restant dépourvue de colle.

14. Emballage (2) selon l'une quelconque des revendications 13 à 7 et 11 à 13, **caractérisé en ce que** l'écran (26) n'est pas relié à la feuille de couverture et repose sur les produits contenus dans l'emballage (2), sur une pièce (12) contenue dans l'emballage ou sur des appuis que celui-ci comprend Intérieurement.

## Claims

1. Packaging (2) for sterile products or products intended to be sterilized comprising a tub (4) made of plastic and a cover (16) fixed to the tub (4) so as to seal the latter with an impervious sealing zone, the cover (16) comprising:
- a cover sheet (20) made of plastic, transparent to electron irradiation and light radiation;
- at least one window (22) formed in the cover sheet (20),
- at least one sheet (24) of a selectively impervious material, impervious to contamination by micro-organisms, bacteria and/or a biologically active material while at the same time remaining permeable to a sterilization gas, secured to the said cover sheet (20) and closing off the window (22);
- and a screen (26, 126) which is opaque to at least an electron irradiation passing through the cover sheet or the selectively impervious material, the said screen extending inside the packaging (2) near the cover sheet (20) so as to allow a sterilizing gas, for example ethylene oxide or steam, to enter through the selectively impervious material.

2. Packaging (2) according to Claim 1, **characterized in that** the sheet(s) (24) of selectively impervious material is (are) bonded under the cover sheet (20).

3. Packaging (2) according to Claim 1 or 2, **characterized in that** the selectively impervious material is a material based on filaments of HDPE or some other polymer, bound together by heat and pressure.

4. Packaging (2) according to any one of Claims 1 to 3, **characterized in that** the window (22) extends near the upper peripheral edges (14) of the tub (4).

5. Packaging (2) according to any one of Claims 1 to 4, **characterized in that** the screen (26, 126) comprises an assembly of two sheets of selectively impervious material.

6. Packaging (2) according to any one of Claims 1 to 4, **characterized in that** the screen (26, 126) comprises a metallized material or a flexible metal foil.

7. Packaging (2) according to Claim 6, **characterized in that** the screen (26) is a flexible aluminium foil.

8. Packaging (2) according to any one of Claims 1 to 7, **characterized in that** the screen (26, 126) is fixed under the cover sheet (20) along a fixing line (28) or along fixing points.

9. Packaging (2) according to any one of Claims 1 to 5, **characterized in that** the selectively impervious screen (126) extends under the cover sheet (20) and has dimensions allowing its peripheral part (126a) to be sandwiched between the cover sheet (20) and the upper peripheral edge (14) of the tub (4).

10. Packaging (2) according to any one of Claims 1 to 7, **characterized in that** the impervious screen (126) extends under the cover sheet (20) and has dimensions allowing its peripheral part (126a) to be sandwiched between the cover sheet (20) and the upper peripheral edge (14) of the tub (4) on the one hand, and on the other hand has orifices for fluidic communication with at least one window (22).

11. Packaging (2) according to any one of Claims 1 to 10, **characterized in that** the cover (16) comprises usage indicators (30) making it possible to identify and indicate the type of sterilization and/or decontamination to which the said packaging (2) has been subjected.

12. Packaging (2) according to any one of Claims 1 to 11, **characterized in that** the tub (4) is made of a plastic that is transparent to light radiation.

13. Packaging (2) according to any one of Claims 1 to 12, **characterized in that** the sheet (24) of selectively impervious material has a peripheral connecting zone (24a) over which an adhesive compatible with high-temperature sterilization is continuously spread, and a central zone (24b) which remains devoid of adhesive.

14. Packaging (2) according to any one of Claims 1, 3 to 7 and 11 to 13, **characterized in that** the screen (26) is not connected to the cover sheet and rests on the products contained in the packaging (2), on a piece (12) contained in the packaging or on supports that the latter comprises on its inside.

## Patentansprüche

1. Verpackung (2) für sterile oder zu sterilisierende Produkte, mit einer Schachtel (4) aus Kunststoff und einem Deckel (16), der auf der Schachtel (4) derart befestigt ist, dass er Letztere durch eine dichte Verschließzone verschließt, wobei der Deckel (16) aufweist:
- eine Abdeckfolie (20) aus Kunststoff, die gegenüber elektronischer Einstrahlung und Lichtstrahlung transparent ist;
- zumindest ein Fenster (22), das in der Abdeckfolie (20) ausgespart ist,
- zumindest eine Folie (24) aus einem gegenüber der Kontamination durch Mikroorganismen, Bakterien und/oder ein biologisch aktives Material selektiv dichten Material, das dabei gegenüber einem Sterilisiergas oder Dekontaminiergas durchlässig bleibt, wobei die Folie (24) mit der Abdeckfolie (20) fest verbunden ist und das Fenster (22) verschließt;
- und eine Abschirmung (26, 126), die gegenüber zumindest einer durch die Abdeckfolie oder das selektiv dichte Material hindurchgehende elektronische Einstrahlung undurchlässig ist, wobei sich die Abschirmung im Inneren der Verpackung (2) in Nähe der Abdeckfolie (20) erstreckt, derart, dass das Eindringen eines Sterilisiergases, beispielsweise Ethylenoxid oder Wasserdampf durch das selektiv dichte Material hindurch ermöglicht wird.

2. Verpackung (2) nach Anspruch 1, **dadurch gekennzeichnet, dass** die Folie(n) (24) aus selektiv dichtem Material unter die Abdeckfolie (20) geklebt ist (sind).

3. Verpackung (2) nach Anspruch 1 oder 2, **dadurch gekennzeichnet, dass** das selektiv dichte Material ein Material auf der Basis von Filamenten aus PEHD oder einem anderen Polymer ist, die mittels Wärme und Druck untereinander verbunden sind.

4. Verpackung (2) nach einem der Ansprüche 1 bis 3, **dadurch gekennzeichnet, dass** das Fenster (22) sich in Nähe der oberen Umfangsränder (14) der Schachtel (4) erstreckt.

5. Verpackung (2) nach einem der Ansprüche 1 bis 5, **dadurch gekennzeichnet, dass** die Abschirmung (26, 126) eine Anordnung aus zwei Folien aus selektiv dichtem Material aufweist.

6. Verpackung (2) nach einem der Ansprüche 1 bis 4, **dadurch gekennzeichnet, dass** die Abschirmung (26, 126) ein metallisiertes Material oder eine biegsame Metallfolie aufweist.

7. Verpackung (2) nach Anspruch 6, **dadurch gekennzeichnet, dass** die Abschirmung (26) eine biegsame Aluminiumfolie ist.

8. Verpackung (2) nach einem der Ansprüche 1 bis 7, **dadurch gekennzeichnet, dass** die Abschirmung (26, 126) unter der Abdeckfolie (20) entlang einer Befestigungslinie (128) oder entlang von Befestigungspunkten befestigt ist.

9. Verpackung (2) nach einem der Ansprüche 1 bis 5, **dadurch gekennzeichnet, dass** sich die selektiv dichte Abschirmung (126) unter der Abdeckfolie (20) erstreckt und Abmessungen aufweist, die es ermöglichen, dass ihr umfänglicher Teil (126a) zwischen der Abdeckfolie (20) und dem oberen Umfangsrand (14) der Schachtel (4) in Sandwich genommen ist.

10. Verpackung (2) nach einem der Ansprüche 1 bis 7, **dadurch gekennzeichnet, dass** sich die dichte Abschirmung (126) unter der Abdeckfolie (20) erstreckt und Abmessungen aufweist, die es ermöglichen, dass ihr umfänglicher Teil (126a) zwischen der Abdeckfolie (20) und dem oberen Umfangsrand (14) der Schachtel (4) einerseits in Sandwich genommen ist, und andererseits Öffnungen aufweist, die in Fluidkommunikation mit zumindest einem Fenster (22) stehen.

11. Verpackung (2) nach einem der Ansprüche 1 bis 10, **dadurch gekennzeichnet, dass** der Deckel (16) Gebrauchsindikatoren (30) aufweist, die es ermöglichen, den Zustand der Sterilisation und/oder Dekontamination zu identifizieren und anzuzeigen, denen die Verpackung (2) unterworfen worden ist.

12. Verpackung (2) nach einem der Ansprüche 1 bis 11, **dadurch gekennzeichnet, dass** die Schachtel (4) aus einem gegenüber Lichtstrahlung transparenten Kunststoff ist.

13. Verpackung (2) nach einem der Ansprüche 1 bis 12, **dadurch gekennzeichnet, dass** die Folie (24) aus selektiv dichtem Material eine umfängliche Verbindungszone (24a), auf der ein mit einer Sterilisierung bei hoher Temperatur kompatibler Klebstoff durchgehend verteilt ist, und eine mittige Zone (24b) aufweist, die frei von dem Klebstoff bleibt.

14. Verpackung (2) nach einem der Ansprüche 1, 3 bis 7 und 11 bis 13, **dadurch gekennzeichnet, dass** die Abschirmung (26) nicht mit der Abdeckfolie verbunden ist und auf den in der Verpackung (2) enthaltenen Produkten, auf einem in der Verpackung enthaltenen Stück (12) oder auf Auflagen, die diese im Inneren aufweist, ruht.
